# EUROPEAN PATENT APPLICATION

(11) **EP 4 413 944 A1**
(43) Date of publication of application: **14.08.2024**
(21) Application number: 23156090.5
(22) Date of filing: 10.02.2023
(51) Int. Cl.: A61B 46/10, A61B 34/20, A61B 17/00

(54) **STERILE HANDHELD INSTRUMENT ASSEMBLY FOR USE IN A SURGICAL ENVIRONMENT**

(71) Applicant: Stryker European Operations Limited, Carrigtwohill, Co. Cork T45 HX08 (IE)
(72) Inventor: PFEIL, Antoine, 67530 Klingenthal, Boersch (FR); SCHAAL, Frederik, 10539 Berlin (DE)
(74) Representative: Röthinger, Rainer

(57) **Abstract**

The present disclosure provides a handheld instrument assembly (100) for use in a surgical environment. The assembly (100) comprises a non-sterile handle (14) and a treatment tool (28) extending from the handle (14) in a distal direction of the handheld instrument assembly (100). The treatment tool (28) comprises a releasable portion (22) which is releasably attached or attachable to the handle (14) or to an adjoining portion (16) of the treatment tool (28). The assembly (100) further comprises a sterile envelope (20) enveloping the non-sterile handle (14) for being gripped by a user of the handheld instrument assembly (100). The present disclosure also provides a cover assembly (12), a handheld instrument (10), and methods of assembling handheld instrument assemblies for use in a surgical environment.

## Description

### Technical Field

The present disclosure relates to handheld instrument assemblies for use in a surgical environment, such as for example pointer instruments, in particular navigation pointer instruments, and the specific demands due to the usage in a sterile surgical environment. The present disclosure also provides a cover assembly for use with a handheld instrument, a handheld instrument for use with a cover assembly, and a method of assembling a handheld instrument assembly for use in a surgical environment

### Background

Sterility is of paramount importance for surgical instruments touching anatomical tissues after an incision, to minimize the risk of infections.

Current solutions for providing sterility encompass single-use sterile instruments or re-usable sterile instruments. Single-use sterile instruments are sterilized after production and sealed in a sterile manner. They are thrown away after use, i.e. are disposables. Gamma sterilization is a typical sterilization method for single-use parts. The cleaning, disinfection and sterilization of re-usable sterile instruments is realized directly in the hospital. Autoclave and ETO (Ethylene Oxide) sterilization are the most common sterilization procedures. Alternatively, the re-usable surgical instrument may be covered with a sterile bag for providing sterility. However, care has to be taken that the sterile cover is not damaged, for example by sharp geometries of the surgical instrument. Furthermore, the function and the handling of the surgical instrument may be impaired by the sterile cover.

Most surgical instruments are offered as re-usable surgical instruments due to the high costs which would arise when offering them as disposable items. However, the sterilization of the re-usable instruments adds significant constraints and costs, both on the hospital-side and on the manufacturer-side of the respective surgical instrument.

On the hospital-side, the sterilization of instruments is cost- and time-intensive, as the overall sterilization chain requires human resources and maintenance work. Instrument management may also be a challenge, as each specific instrument set should be readily prepared for the planned procedure. If, by accident, a surgical instrument is defective or is contaminated (e.g., when the surgical instrument falls on the floor), the whole procedure must be aborted or postponed.

On the manufacturer-side, the sterilization of surgical instruments is related to significant development time and costs. On the testing side, the sterilization validation requires intense testing, and this process often lasts more than one year. Additionally, on the R&D side, the sterilization adds significant design constraints. To ensure compatibility with autoclave procedures, the surgical instrument has to be sealed properly with a dedicated housing, which has an impact on the instrument compactness and weight, which are very important criteria for instrument handling. Due to the high temperatures and pressures used during sterilization, this process also leads to instrument wearing and is the main contributor to the instrument's limited lifetime.

### Summary

The present disclosure provides one or more solutions to the above-mentioned and other challenges.

According to a first aspect, a handheld instrument assembly for use in a surgical environment is provided. The assembly comprises a non-sterile handle, a treatment tool extending from the handle in a distal direction of the handheld instrument assembly, wherein the treatment tool comprises a releasable portion which is releasably attached or attachable to the handle or to an adjoining portion of the treatment tool, and a sterile envelope enveloping the non-sterile handle for being gripped by a user of the handheld instrument assembly.

The handheld instrument assembly may be any handheld instrument which is adapted to be used in a surgical environment. The handheld instrument assembly may be a general pointer instrument, a navigation pointer instrument, an ultrasound device, a biopsy device, a cutting device, or an endoscopic device. For being adapted to be used in a surgical environment, the respective instrument assembly has to fulfil the sterility requirement.

The handle may be any part of the handheld instrument assembly which is configured to be grippable by a user for holding the handheld instrument assembly. The handle may be cleaned and disinfected. The handle may be a re-usable item or a disposable item.

The treatment tool may be the part of the handheld instrument assembly which defines its function. Thus, the configuration of the treatment tool depends on the intended use and function of the respective handheld instrument assembly. In the example of the pointer instrument, the treatment tool may be the pointed tip, and in the example of the cutting device, the treatment tool may be the cutting edge. The treatment tool extends from the handle in a direction away from a user of the handheld instrument assembly holding the assembly. The treatment tool, in the use of the handheld instrument assembly, may be close to the patient's body. The treatment tool may comprise different, separate portions, which, when assembled, define the treatment tool. The different, separate portions may be disposable and/or re-usable items. In particular, the treatment tool may comprise disposable and re-usable portions.

The releasable portion of the treatment tool may be the treatment tool itself which is releasably attached or attachable to the handle. As an example, the treatment tool may consist of the portion which is releasably attached or attachable to the handle.

The releasable portion may be a portion of the treatment tool which may be releasably attached or attachable to an adjoining portion of the treatment tool or to another part of the treatment tool. The releasable connection may be any known releasable connection. The releasable connection may be a mechanical releasable connection, for example by means of cooperating mechanical features of the portions to be connected. For example, the mechanical releasable connection may be a plug connection. In addition or in the alternative, the releasable connection may be a magnetic connection, for example. The releasable connection may be realized by a releasable adhesive connection, for example by means of an adhesive tape.

The releasable connection may be or comprise a locking connection which secures the connected portions in place relative to each other so as to be releasably locked. Thus, for example, in case the releasable connection comprises a mechanical connection, the mechanical connection may comprise mechanical interlocking features.

The releasable portion may in some variants also be covered by the sterile envelope. In other variants, it may not be covered by the sterile envelope.

The handle and the treatment tool may define a handheld instrument. The treatment tool may be also releasably connected to the handle.

The sterile envelope may have, or be configured to be formed to have, any shape and property adapted to envelope the handle. For example, the sterile envelope enveloping the non-sterile handle may be flexible. Thus, the sterile envelope may closely hug the contour of the handle which is covered by the sterile envelope. For example, the sterile envelope may be a drape or a foil or a bag. The sterile envelope may be transparent with respect to the spectrum of the environmental light.

The sterile envelope may further envelope other parts or portions of the handheld instrument assembly. The sterile envelope may also envelope the overall handheld instrument.

The sterile envelope may a disposable item or a re-usable item. In case the sterile envelope is a re-usable item, it has to be reprocessed, e.g., cleaned, disinfected and sterilized, before being re-used.

In some implementations of this first aspect, the assembly may comprise one or more tracking elements, wherein the sterile envelope may be arranged to envelop the one or more tracking elements. The one or more tracking elements may be provided anywhere on or at the assembly, for example on the handle. In particular, in variants of the some implementations, the one or more tracking elements comprise one or more active tracking elements, wherein the sterile envelope is transparent with regard to the at least part of the spectrum of the light emitted by the one or more active tracking elements. The one or more tracking elements may be only one or more active tracking elements.

In further some implementations of this aspect, the releasable portion may form a distal portion of the treatment tool and may be releasably attached or attachable to a proximal portion of the treatment tool, wherein the proximal portion of the treatment tool may be enveloped by the sterile envelope. The distal portion is located further away from a user gripping the handheld instrument assembly than the proximal portion. The distal portion may be located nearer to a patient's body than the proximal portion during surgery. The distal and proximal portions of the treatment tool may form the treatment tool. The proximal portion of the treatment tool may be releasably connected to the handle and/or may be non-sterile.

In some variants to this first aspect, the releasable portion may be fixed to or integral with the sterile envelope. The releasable portion may be permanently fixed to the sterile envelope. The releasable portion may be part of a cover assembly also comprising the sterile envelope. The cover assembly may be a disposable component.

In further some variants of this aspect, the releasable portion may form the treatment tool which is releasably attached or attachable at a proximal end to the handle enveloped by the sterile envelope, wherein the treatment tool may not be enveloped by the sterile envelope. In these other implementations, the single component that is enveloped by the sterile envelope may be the handle, optionally the handle including one or more tracking elements. The overall treatment tool may be sterile. The treatment tool may be a re-usable component or a single component. Furthermore, the handle may be configured to releasably attach a plurality of different treatment tools. The different treatment tools may have different geometries and/or sizes. The releasable portion that forms the treatment tool may be also fixed to or integral with the sterile envelope and releasably attached or attachable to the handle, together with the sterile envelope, before the sterile envelope is everted to cover the handle.

In some implementations of this first aspect, the releasable portion may form a sharp tip of the treatment tool. The releasable portion with the sharp tip may not be covered by the sterile envelope. The sharp tip may be the sharp tip of a pointer instrument. The sharp tip may be the sharp edge of a cutting instrument or a sharp grip of gripping instrument.

In further implementations of this aspect, the releasable portion may be sterile. The sterile releasable portion may be a re-usable component or a sterile disposable item. The sterile releasable portion may not be covered by the sterile envelope. The sterile releasable portion may be attached to or be integral with the sterile envelope.

In some implementations of this aspect, the releasable portion may be releasably attached or attachable by a plug connection. The releasable portion may be form-fittingly connected. The plug connection may comprise a locking mechanism, for example by means of a spring force securing the releasable portion in place relative to the connected portion or part.

In some implementations, the releasable portion may be a blunt shaped cap hiding a sharp distal end of the treatment tool, wherein the treatment tool with the blunt shaped cap may be enveloped by the sterile envelope. The blunt shaped cap may be sized and shaped so as to be able to be put on the sharp distal end for covering the sharp distal end. The blunt shaped cap may be a hollow cone or a hollow truncated cone. The cone or truncated cone may be configured with a round-shaped tip at the small diameter end of the cone or truncated cone. The blunt shaped cap may be fixed to the distal end of the treatment tool by means of an adhesive connection. The adhesive connection may be a releasable connection. The blunt shaped cap may be configured adhesive or comprise an adhesive tape at an interface to the distal end of the treatment tool. In addition or in the alternative, the distal end of the treatment tool may be configured adhesive or comprise an adhesive tape. The blunt shaped cap may be attached or attachable to the distal end by any kind of connection means, in particular by any kind of releasable connection means. The connection means may be provided at at least one of the blunt shaped cap and the distal end of the treatment tool.

In some implementations, the sterile envelope may have, or may be able to be formed to define, an opening for inserting the non-sterile handle and, optionally, the treatment tool or a proximal portion of the treatment tool, wherein the opening may be sealingly closed in an assembled state of the handheld instrument assembly (e.g., in a bag-like manner). The opening may be the single opening of or formed by the sterile envelope. The closing may be performed by connecting portions of the sterile envelope surrounding the opening. The connection may be established by means of an elastic tightly closing the opening or an adhesive joint, for example.

In some implementations, the sterile envelope may have an inner non-sterile surface side and an outer sterile surface side. The outer sterile surface side may be for being gripped by the user of the handheld instrument assembly. The inner non-sterile surface side may delimit a cavity housing non-sterile parts of the assembly. The outer sterile surface side may ensure the sterility of the assembly required for use in a surgical environment. In other implementations, also the inner surface side may be sterile.

In some implementations, at least one of the releasable portion and the sterile envelope may be a disposable item. The disposable item may be sterile, for example by means of a gamma sterilization. The disposable item may be replaced after a surgery operation with another disposable item.

In some implementations, the handheld instrument assembly may be a pointer instrument assembly. The pointer instrument assembly may be a navigation pointer instrument assembly. The sharp tip of the navigation pointer instrument assembly may be hidden by the blunt shaped cap or may be located outside of the sterile envelope so that there is no risk that the sharp tip might damage the sterile envelope. The navigation pointer instrument assembly may comprise one or more tracking elements, in particular one or more active tracking elements. The one or more tracking elements may be provided on the handle or in a spatially fixed relationship with the handle. The navigation pointer instrument assembly may be used for defining position information of dedicated points at the patient's body (e.g., for establishing a registration between the patient's body and image data of the patient's body taken beforehand.

According to a second aspect, the present disclosure provides a cover assembly for use with a handheld instrument. The cover assembly comprises an envelope having a sterile outer surface side for being gripped by a user of the handheld instrument, wherein the envelope is configured to envelop the handheld instrument. The cover assembly further comprises a distal treatment portion being fixed to or integral with the envelope so as to protrude from the sterile outer surface side of the envelope, wherein the distal treatment portion is configured to be releasably attachable to a proximal treatment portion or a handle of the handheld instrument so as to form a treatment tool.

The handheld instrument may be a surgical handheld instrument. The envelope may be a flexible envelope. The distal treatment portion may be permanently fixed to the envelope. The distal treatment portion attached to or integral with the sterile envelope may be one of two or more portions of the treatment tool, or it may constitute the entire treatment tool. The distal treatment portion may be sterile. The cover assembly may be part of the handheld instrument assembly according to the first aspect described herein and/or according to one or some implementations of this first aspect described herein. The envelope may be configured as the sterile envelope described above with respect to the handheld instrument assembly according to a first aspect of the present disclosure and the implementations thereof. In some implementations of the cover assembly, the distal treatment portion of the sterile envelope may comprise a tip configured to form a distal end of the treatment tool. The tip may be a sharp tip. Since the tip protrudes from the outer surface of the envelope, the tip is in some variants not covered by the envelope.

According to a third aspect of the present disclosure, a handheld instrument for use in a surgical environment and for use with a cover assembly is provided. The cover assembly has a sterile envelope being configured to envelop the handheld instrument, and a distal treatment portion is fixed to or integral with the envelope. The handheld instrument comprises a non-sterile handle and a non-sterile proximal treatment portion extending from the handle in a distal direction of the handheld instrument. The proximal treatment portion is configured to be releasably attachable to the distal treatment portion of the cover assembly so as to form a treatment tool.

The handheld instrument may be configured to be used in combination with a cover assembly according to the third aspect of the present disclosure or an implementation thereof. The proximal treatment portion of the handheld instrument may not have a sharp tip. The proximal treatment portion may be configured to have a rounded contour, i.e. does not comprise any sharp edges, at least after being connected to the distal treatment portion. The handheld instrument may be a conventional pointer instrument or navigation pointer instrument without the provision of a sharp tip at a distal end of the pointer instrument. The sharp tip may be part of the distal treatment portion of the envelope. The handheld instrument when assembled with the cover assembly may be a handheld instrument assembly according to the first aspect or to one or some of the implementations thereof. The sterile envelope may be flexible. The distal treatment portion may be sterile. The handheld instrument may comprise one or more tracking elements, in particular one or more active tracking elements. The sterile envelope may be configured to envelop the overall handheld instrument.

According to a fourth aspect of the present disclosure, a method of assembling a handheld instrument assembly for use in a surgical environment is provided. The method comprises the steps of providing a cover assembly including a sterile envelope having, or being able to be formed to define, an opening and having a first, sterile envelope surface side, and a distal treatment portion being fixed to or integral with the sterile envelope so as to protrude from the first, sterile envelope surface side into a hollow space delimited by the first, sterile envelope surface side, providing a handheld instrument comprising a non-sterile handle and, optionally, a non-sterile proximal treatment portion extending from the handle in a distal direction of the handheld instrument, releasably connecting the cover assembly and the handheld instrument by releasably connecting a proximal end of the distal treatment portion of the cover assembly to a distal end of the non-sterile proximal treatment portion of the handheld instrument or to the handle of the handheld instrument, the proximal end of the distal treatment portion of the cover assembly being accessible from a second envelope surface side opposite the first, sterile envelope surface side, and, after having established the releasable connection, everting the sterile envelope so that the first, sterile envelope surface side becomes an outer, sterile envelope surface side and the second envelope surface side becomes in inner surface side delimiting a hollow space receiving the non-sterile handle of the handheld instrument and, optionally, the non-sterile proximal treatment portion of the handheld instrument. The method further comprises the step of sealingly closing the opening of the flexible envelope.

The chronology of the method steps is not limited to the described sequence of the method steps if not explicitly excluded. In the disclosed first method, the envelope may be flexible. Furthermore, the distal treatment portion may be permanently fixed to the envelope. The assembling method may result in a handheld instrument assembly according to the first aspect or to one or some of the implementations thereof. In particular, the assembled handheld instrument assembly may comprise a distal treatment portion that is not covered by the sterile envelope, whereas the proximal treatment portion and the handle is covered with the sterile envelope. The distal treatment portion may comprise a sharp tip.

According to a fifth aspect of the present disclosure, another method of assembling a handheld instrument assembly for use in a surgical environment is provided. The other method comprises the steps of providing a transparent sterile envelope having, or being able to be formed to define, an opening, providing a handheld instrument having a non-sterile handle with one or more tracking elements and a sterile treatment tool being structurally separate and disconnected from the non-sterile handle, wherein a proximal end of the treatment tool and a distal end of the handle are configured to be releasably attachable with each other, enclosing the non-sterile handle having the one or more tracking elements with the transparent sterile envelope, releasably connecting the enclosed handle and the treatment tool by releasably connecting the proximal end of the treatment tool and the distal end of the enclosed handle, and sealingly closing the opening of the sterile envelope. Again, the chronology of the method steps is not limited to the described sequence of the method steps if not explicitly excluded. In the other method, the envelope may be flexible. The one or more tracking elements may be or comprise active tracking elements. The sterile envelope may be at least transparent with regard to the spectrum of the emitted light of the one or more active tracking elements. The other assembling method may result in a handheld instrument assembly according to the first aspect or to one or some of the implementations thereof. In particular, the assembled handheld instrument assembly may comprise a treatment tool that is not covered by the sterile envelope, whereas the handle with the one or more tracking elements is covered with the sterile envelope. The treatment tool may comprise a sharp distal tip.

According to a sixth aspect of the present disclosure, a still further method of assembling a handheld instrument assembly for use in a surgical environment is provided. The still further method comprises the steps of providing a sterile envelope having, or being able to be formed to define, an opening, providing a non-sterile handheld instrument having a handle and a treatment tool, the treatment tool having a tip portion extending from the handle in a distal direction of the handheld instrument and having a blunt shaped cap structurally separate from and disconnected from the tip portion, the tip portion having a sharp tip at a distal end, attaching the blunt shaped cap to the distal end of the tip portion so as to hide the sharp tip, enclosing the non-sterile handheld instrument with the attached blunt shaped cap with the sterile envelope, and sealingly closing the opening of the sterile envelope.

The chronology of the method steps is not limited to the described sequence of the method steps if not explicitly excluded. In the disclosed further method, the envelope may be flexible. The non-sterile handheld instrument may comprise one or more tracking elements, in particular one or more active tracking elements. The sterile envelope may be at least transparent with regard to the spectrum of the emitted light of the one or more active tracking elements. The further assembling method may result in a handheld instrument assembly according to the first aspect or to one or some of the implementations thereof. In particular, the assembled handheld instrument assembly may comprise a handheld instrument that is completely covered with the sterile envelope.

### Brief Description of the Drawings

Further details, advantages and aspects of the present disclosure will become apparent from the following embodiments taken in conjunction with the drawings, wherein:
Figs. 1A and 1B schematically show an embodiment of a handheld instrument assembly comprising a handheld instrument and a cover assembly according to the present disclosure;
Figs. 2A and 2B schematically show a method of assembling the handheld instrument assembly of Figs. 1A and 1B;
Fig. 3 schematically shows another embodiment of a handheld instrument assembly according to the present disclosure;
Figs. 4A-4C schematically show a method of assembling the handheld instrument assembly of Fig. 3;
Fig. 5 schematically shows a plurality of different tip portions that may be part of a handheld instrument assembly of Fig. 3;
Fig. 6 schematically shows still another embodiment of a handheld instrument assembly according to the present disclosure;
Figs. 7A and 7B schematically show a method of assembling the handheld instrument assembly of Fig. 6;
Fig. 8 shows a flow chart of a method of assembling the handheld instrument assembly of Figs. 1A and 1B;
Fig. 9 shows a flow chart of a method of assembling the handheld instrument assembly of Fig. 3; and
Fig. 10 shows a flow chart of a method of assembling the handheld instrument assembly of Fig. 6.

### Detailed Description

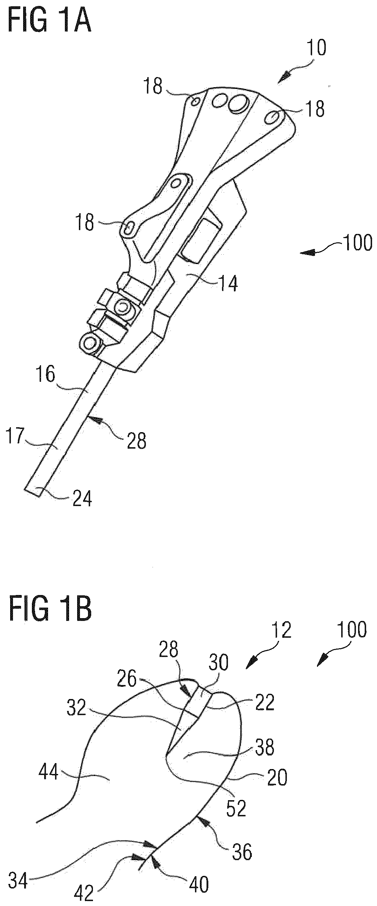

Figs. 1A and 1B of the drawings show components of a handheld instrument assembly 100 according to an embodiment of the present disclosure in an unassembled state. The handheld instrument assembly 100 comprises a handheld instrument 10 as shown in Fig. 1A and a cover assembly 12 as shown in Fig. 1B. The handheld instrument 10 of Fig. 1A forms a capital component of the handheld instrument assembly 100 and is a non-sterile re-usable component of the handheld instrument assembly 100. The cover assembly 12 is a sterile disposable item.

The handheld instrument assembly 100 of Figs. 1A and 1B is a surgical navigation pointer instrument. It will be apparent that the assembly 100 could be any other surgical instrument

In the present application, the term "proximal" is used for describing features that are located proximal, i.e. near, to a user of the handheld instrument assembly 100, and the term "distal" is used for describing features that are located distal to, i.e. further away from, a user of the handheld instrument assembly 100. In other words, proximal features are located nearer to a user than distal features and, vice versa, distal features are located further away from a user than proximal features. Correspondingly, the term "in a distal direction" describes the direction away from a user of the handheld instrument assembly 100, and the term "in a proximal direction" describes the direction towards a user of the handheld instrument assembly 100.

The handheld instrument 10 of Fig. 1A comprises a non-sterile handle 14 configured to be gripped by a user of the handheld instrument assembly 100 such as a surgeon and a non-sterile proximal treatment portion 16 extending from the handle 14 in a distal direction of the handheld instrument assembly 100. In the present example, the proximal treatment portion 16 is fixedly attached to the handle 14. In other examples, the proximal treatment portion 16 may be releasably attached to the handle 14.

The proximal treatment portion 16 is a generally hollow tube 17. In other examples, the proximal treatment portion 16 may be a solid construction or a construction being partly hollow and partly solid. The handle 14 comprises one or more tracking elements 18, in particular one or more active tracking elements 18. More particularly, in the present example, the handle 14 comprises a plurality of active tracking elements 18 (e.g., light emitting diodes, LEDs, configured to emit in the visible of infrared spectrum) for enabling a tracking of the position of the pointer instrument. The tracking elements 18 may be configured to be removable from the handle 14. The tracking elements 18 in combination form an instrument tracker that is to be detected by a tracking camera of a surgical tracking system.

The capital component of Fig. 1A is configured to be combined with, in particular to be mounted to, the cover assembly 12 of Fig. 1B so as to form the handheld instrument assembly 100. The cover assembly 12 of Fig. 1B comprises a sterile envelope 20 and a metallic tip portion 22 that is configured to cooperate with a distal end 24 of the hollow tube forming the proximal treatment portion 16. In the present example, the sterile envelope 20 is a drape and is transparent. In particular, the sterile envelope 20 may be transparent with regard to the electromagnetic spectrum of the ambient light of a surgical environment. In the present embodiment with the active trackers 18, the sterile envelope 20 is at least transparent with regard to part of the electromagnetic spectrum of the light emitted by the active tracking elements 18.

The metallic tip portion 22 forms a distal treatment portion 26. The distal treatment portion 26 and the proximal treatment portion 16 assembled together form a treatment tool 28. In the present example of the navigation pointer instrument, the treatment tool 28 is the pointer.

The metallic tip portion 22 is fixed to the sterile envelope 20. The metallic tip portion 22 is also sterile. In the present example, the metallic tip portion 22 is permanently fixed to the sterile envelope 20, e.g., by gluing or use of any other adhesive. The metallic tip portion 22 has a proximal portion 30 that is tube-shaped and has a substantially constant outer diameter, and a distal portion 32 that tapers at its distal end so as to form a sharp tip 52. The metallic tip portion 22 is configured to be releasably attachable to the distal end 24 of the proximal treatment portion 16. In particular, in the present example, the outer diameter of the proximal portion 30 of the tip portion 22 is slightly smaller than an inner diameter of the distal portion 32 of the hollow tube 17 forming the proximal treatment portion 16, so that the metallic tip portion 22 is configured to be insertable into the hollow tube 17 with its proximal portion 30. Furthermore, in the present example, the hollow tube 17 and the tip portion 22 comprise mutually cooperating locking features (not shown here) forming a locking mechanism which allows the metallic tip to be locked to and such being secured in place to the hollow tube forming the proximal treatment portion of the treatment tool. In other examples, only one of the hollow tube 17 and the tip portion 22 may comprise one or more locking features.

The sterile envelope 20 has at least one sterile surface 34. The sterile tip portion 22 is fixed to the sterile envelope 20 so as to protrude from and extend away from the at least one sterile surface 34 of the envelope 20. The other surface 36 of the envelope 20 may be also sterile or may be non-sterile. In the present example, the other surface 36 is non-sterile.

In Fig. 1B, the sterile envelope 20 is shaped to form a hollow space 38 into which the metallic tip portion 22 protrudes and which is delimited by the sterile surface 34 of the envelope 20. Thus, the sterile tip portion 22 faces the sterile surface 34 of the envelope 20. The other surface 36 forms in the state of Fig. 1B an outer surface side 40 and is, in the present example, a non-sterile outer surface side 40, which may be touched by a user without impairing the sterility of the inner surface side 42 and the metallic tip portion 22. The sterile envelope is further shaped to form an opening 44.

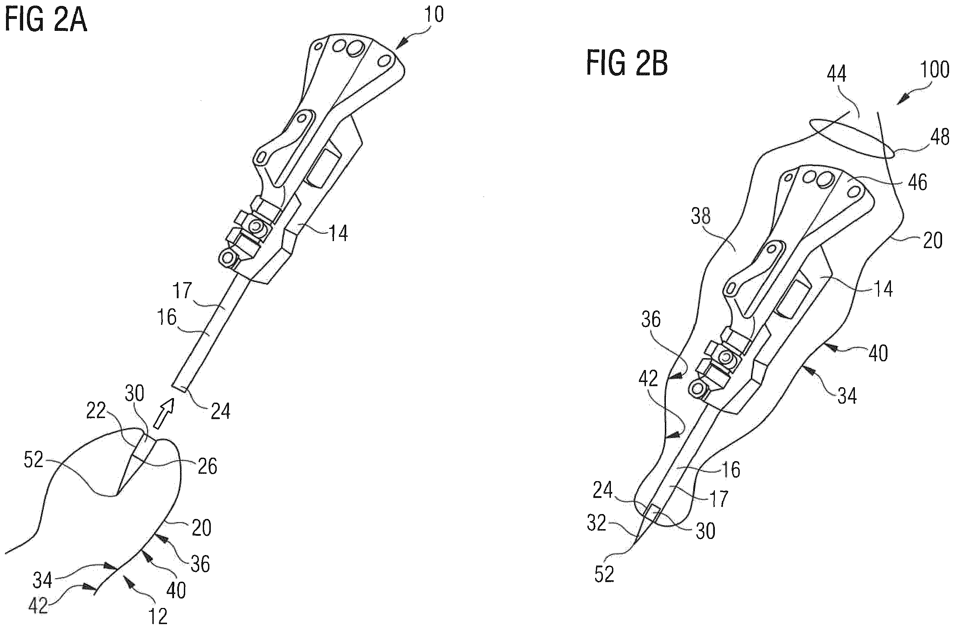

Figs. 2A and 2B illustrate a method of assembling a handheld instrument assembly 100. In Figs. 2A and 2B, the handheld instrument assembly 100 is the navigation pointer instrument of Figs. 1A and 1B. The method may be performed in a sterile environment, for example in the operating room.

In a first step, the re-usable capital component of the handheld instrument assembly 100 as shown in Fig. 1A is provided, comprising the handle 14 and the proximal treatment portion 16 fixed thereto. If the re-usable capital component is not already cleaned and disinfected, providing the re-usable capital component encompasses cleaning and disinfecting the capital component. The re-usable capital component is not sterilized.

In a second step, the sterile cover assembly 12 comprising the sterile envelope 20 and the sterile distal treatment portion 26 are provided. The sterile cover assembly 12 is provided in the state as shown in Fig. 1B, with the sterile envelope 20 forming a hollow space 38 which is delimited by the first sterile surface side 34 of the envelope 20 and with the distal treatment portion 26 protruding into the hollow space 38. In the state shown in Fig. 2A, the first sterile surface side 34 forms in an inner surface side 42 and the second non-sterile surface side 36 of the envelope 20 opposite to the first surface side 34 forms an outer surface side 40.

In a subsequent third step, the metallic tip portion 22 forming the distal treatment portion 26 is plugged into the hollow tube 17 forming the proximal treatment portion 16 so as to form the treatment tool 28 of the handheld instrument assembly 100. In particular, the proximal portion 30 of the metallic tip portion 22 forming the distal treatment portion 26 is inserted into the hollow tube 17 of the proximal treatment portion 16 so as to releasably connect the handheld instrument 10 and the cover assembly 12 with each other. An optional locking mechanism (not shown here) provided in the proximal treatment portion 16 and the distal treatment portion 26 ensures that the distal treatment portion 26 stays securely in place relative to the capital component.

As is seen in Fig. 2A, for plugging the distal treatment portion 26 into the proximal treatment portion 16, the user (e.g., a surgical assistant) grips the cover assembly 12 at its outer, non-sterile surface side 36, 40 while maintaining the sterile environment in the hollow space 28 formed by the sterile surface side 34 of the sterile envelope 20. Furthermore, the proximal portion 30 of the distal treatment portion 26 is attached to the sterile envelope 20 so as to be accessible from the non-sterile outer surface side 36, 40 of the envelope 20 for being connected to the proximal treatment portion 16.

After having established the releasable connection between the handheld instrument 10 and the cover assembly 12, the sterile envelope 20 is moved over the handheld instrument 10 so as to receive and cover the handheld instrument 10. In particular, the sterile envelope 20 is moved so as to cover and completely envelop the proximal treatment portion 16 and the handle 14 of the handheld instrument 10. In the example of Figs. 2A and 2B, the sterile envelope 20 is inverted so that the formerly inner sterile surface side 34, 42 becomes an outer sterile surface side 34, 40 and the formerly outer non-sterile surface side 36, 40 becomes an inner non-sterile surface side 36, 42.

For rolling out the sterile envelope 20 as described above, the user preferably only touches the outer non-sterile surface side 36, 40, which, after having rolled out, becomes the inner non-sterile surface side 36, 42.

In a last step, the opening 44 of the sterile envelope 20 extending beyond a proximal end 46 of the handheld instrument 10 is closed. The opening 44 may be closed by an elastic 48 (as shown) or an adhesive or zipper (not shown), for example. Preferably, the opening 44 is sealingly closed, for sealing the non-sterile handheld instrument 10 against the sterile environment.

As is seen in Fig. 2B, after having assembled the handheld instrument assembly 100, the overall outer surface side 40 of the assembled handheld instrument assembly 100 facing the sterile environment is a sterile surface side 34. In particular, the distal tip portion 32 and the formerly inner sterile surface side 34, 42 of the envelope 20 form the outer sterile surface side 34, 40 of the assembled assembly 100.

For using the handheld instrument assembly 100, which is a surgical navigation pointer in the present case, the surgeon holds the handle 14 of the handheld instrument assembly which is enclosed by the sterile envelope 20, similarly as he/she would do with a conventional sterilized handheld instrument assembly.

A variant of the example of Figs. 1A, 1B and 2A, 2B, which is not illustrated here, provides a cover assembly 12 that comprises the sterile envelope 20 and the treatment tool 28 attached thereto or being integral thereto. The treatment tool 28 is preferably configured as an integral part and is releasably attachable to the handle 14, together with the sterile envelope 12 and before everting the sterile envelope 12 so as to cover the handle 14.

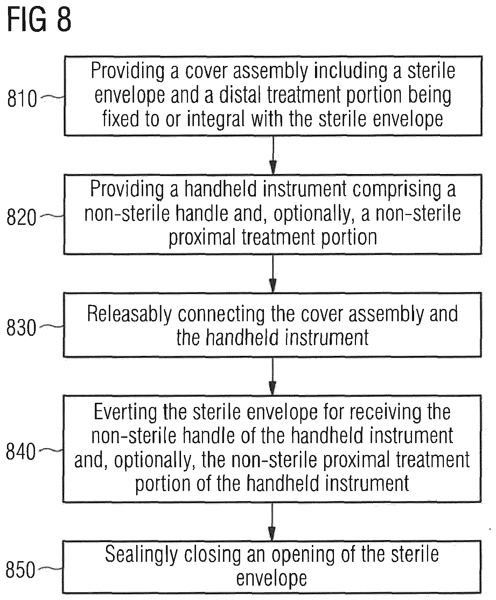

Fig. 8 of the drawings shows a flow chart schematically depicting the process steps of the method described with reference to Figs. 2A and 2B.

In a method step 810, a cover assembly 12 including a sterile envelope 20 and a distal treatment portion 26 being fixed to or integral with the sterile envelope 20 is provided. The sterile envelope 20 has an opening 44 or is able to be formed to define an opening 44. The sterile envelope 20 further has a first, sterile envelope surface side 34. The distal treatment portion 26 is fixed to or integral with the sterile envelope 20 so as to protrude from the first, sterile envelope surface side 24 into a hollow space 38 delimited by the first, sterile envelope surface side 34.

In a method step 820, a handheld instrument 10 comprising a non-sterile handle 14 and, optionally, a non-sterile proximal treatment portion 16 is provided. The proximal treatment portion 16 extends from the handle 14 in a distal direction of the handheld instrument 10.

In a method step 830, the cover assembly 12 and the handheld instrument 10 are releasably connected. In particular, the cover assembly 12 and the handheld instrument 10 are releasably connected by releasably connecting a proximal end 30 of the distal treatment portion 26 of the cover assembly 12 to a distal end 24 of the non-sterile proximal treatment portion 16 of the handheld instrument 10 or to the handle 14 of the handheld instrument 10, wherein the proximal end 30 of the distal treatment portion 26 of the cover assembly 12 is accessible from a second envelope surface side 36 opposite the first, sterile envelope surface side 34.

In a method step 840, the sterile envelope 20 is everted for receiving the non-sterile handle 14 of the handheld instrument 10 and, optionally, the non-sterile proximal treatment portion 24 of the handheld instrument 10. In particular, the sterile envelope 20 is everted so that the first, sterile envelope surface side 34 becomes an outer, sterile envelope surface side 40, 34 and the second envelope surface side 36 becomes in inner surface side 42 delimiting a hollow space 38 receiving the non-sterile handle 14 of the handheld instrument 10 and, optionally, the non-sterile proximal treatment portion 16 of the handheld instrument 10.

In a method step 850, the opening 44 of the sterile envelope 20 is sealingly closed.

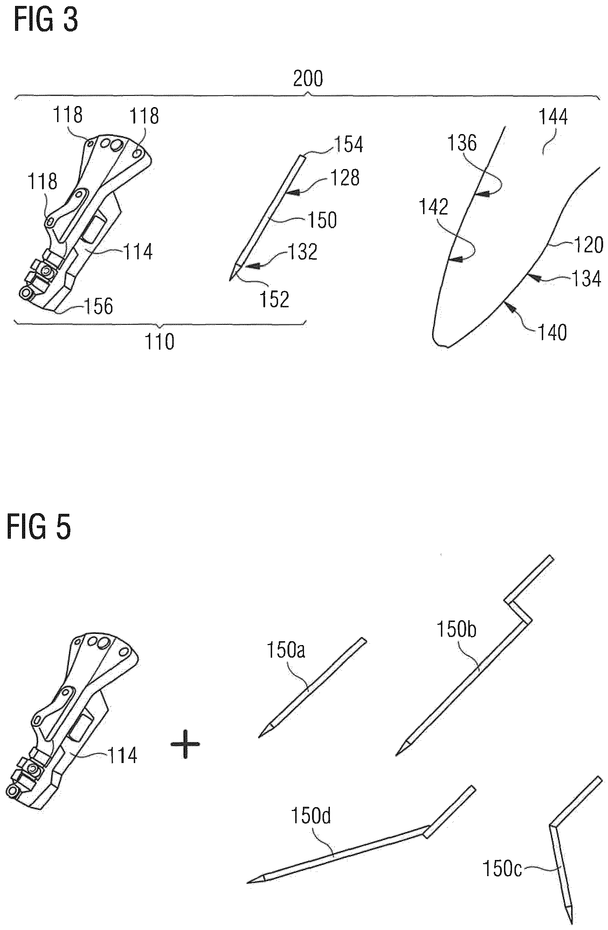

Fig. 3 shows components of another example of a handheld instrument assembly 200 according to the present disclosure. The handheld instrument assembly 200 is a navigation pointer instrument similar as in the example of Figs. 1 and 2. The handheld instrument assembly 200 comprises a handle 114. In the present example, the handle 114 comprises one or more tracking elements 118, in particular one or more active tracking elements 118. The handheld instrument assembly 200 further comprises a (single) tip portion 150, which forms the treatment tool 128 and is releasably attachable to the handle 114 thereby also forming the releasable portion. The handheld instrument assembly 200 also comprises a sterile envelope 120.

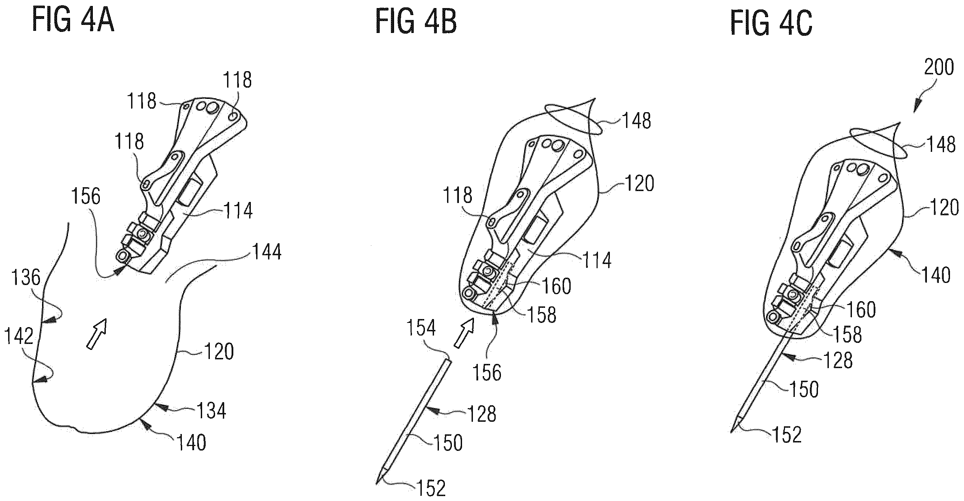

The tip portion 150 forms the pointer. It is cylindrically shaped and has a sharp tip 152 at a distal end 132 of the tip portion 150. The tip portion 150 is releasably attachable at its proximal end 154 to a distal end 156 of the handle 114. For that purpose, in the present example, the material of the handle 114 is partially left out at the distal end 156 thereof so as to form a slot 158 for receiving the proximal end 154 of the tip portion 150, as will be described with reference to Fig. 4B. Further in the present example, the slot 158 is designed to form a locking mechanism for the tip portion 150, as is indicated by the dashed lines 160 in Figs. 4B and 4C.

Other than the treatment tool 28 of Figs. 1A, 1B and 2A, 2B, the present treatment tool 128 of Fig. 3, i.e. the tip portion 150, is formed as a single component and does not comprise a distal portion 26 and a proximal portion 16 releasably attachable to each other. The handle 114 of the present example may be configured as the handle 14 of the example of Figs. 1A, 1B and 2A, 2B.

Likewise, the sterile envelope 120 of the present example may be configured as the sterile envelope 20 described with reference to Figs. 1A, 1B and 2A, 2B, but without the distal treatment portion 26 attached thereto. In particular, the sterile envelope 120 comprises an outer surface side 140 that is a sterile surface side 134. In the present example, the inner surface side 142 is a non-sterile surface side 136. In other examples, the inner surface side 142 is also a sterile surface side 134.

The handle 114 is a re-usable component. However, as will be clear from the following description with regard to Figs. 4, the handle 114 is not sterilized, but only cleaned and disinfected. The tip portion 150 is either a re-usable component or a disposable component. The tip portion 150 is sterile.

The tip portion 150 and the handle 114 form the handheld instrument 110, and, when combined with the sterile envelope 120, form the handheld instrument assembly 200.

Figs. 4A to 4C show an assembly procedure of the handheld instrument assembly 200 of Fig. 3. As is seen in Fig. 4A, the handle 114 with the plurality of active tracking elements 118 and the sterile envelope 120 are provided. The handle 114 and the sterile envelope 120 are structurally separate and disconnected from each other. The handle 114 is not covered by the sterile envelope 120.

As is seen in Figs. 4A and 4B, then, the handle 114 with the tracking elements 118 is covered by the sterile envelope 120. The sterile envelope 120 has a shape so as to have an opening 144 or can be formed so as to have an opening 144 through which the handle 114 is inserted for being completely covered by the sterile envelope 120. The opening 144 may be the sole opening of or formed by the sterile envelope 120.

After having the handle 114 completely enclosed by the sterile envelope 120, the opening 144 is sealingly closed for isolating the non-sterile handle 114 from the sterile environment. For example, the opening 144 is sealingly closed by an elastic 148.

In a next step, the sterile treatment tool 128 is provided. The sterile treatment tool 128 is structurally separate and disconnected from the handle 114 and the sterile envelope 120. The treatment tool 128 formed by the tip portion 150 is inserted into the slot 158 provided in the handle 114 for being releasably connected to the handle 114. In particular, the treatment tool 128 is connected to the handle 114 with a locking mechanism 160. When inserting the tip portion 150 into the slot 158, the sterile envelope 120 is pulled in the slot 158 thereby enveloping the inserted part of the tip portion 150. Thus, the sterile envelope 120 is not damaged thereby preserving the sterile environment for the handle 114 completely enclosed by the sterile envelope 120.

The handheld instrument assembly 200 in an assembled state is illustrated in Fig. 4C. Other than the handheld instrument 100 assembly of Figs. 1 and 2, the treatment tool 128 is not covered by the sterile envelope 120, only the handle 114 is covered and sealingly enclosed by the sterile envelope 120. Thus, in this design variant, the releasable portion 150 having a sharp tip 152 forms the treatment tool 128 which is releasably attached at the proximal end 154 to the handle 114 sealingly enclosed by the sterile envelope 120.

In the design variant according to Figs. 3 and 4A to 4C, the same handle 114 can be used in combination with different tip portion geometries and sizes depending on the targeted anatomy.

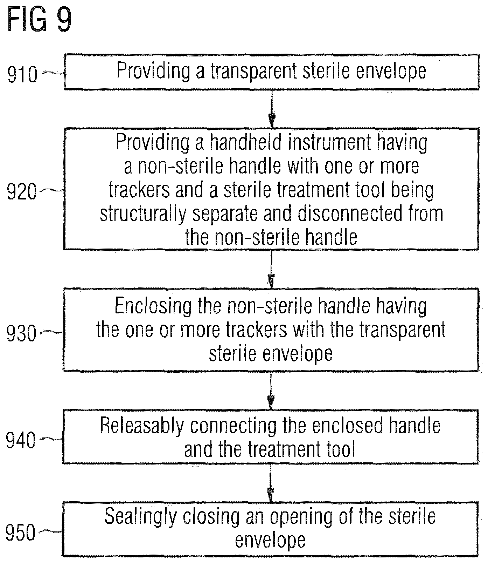

Fig. 9 of the drawings shows a flow chart schematically depicting the process steps of the method described with reference to Figs. 4A to 4C.

In a method step 910, a transparent sterile envelope 120 is provided. The transparent sterile envelope 120 has an opening 144 or is able to be formed to define an opening 144.

In a method step 920, a handheld instrument 110 having a non-sterile handle 114 with one or more tracking elements 118 and a sterile treatment tool 128 being structurally separate and disconnected from the non-sterile handle 114 is provided. A proximal end 154 of the treatment tool 128 and a distal end 156 of the handle 114 are configured to be releasably attachable with each other.

In a method step 930, the non-sterile handle 114 having the one or more tracking elements 128 is enclosed with the transparent sterile envelope 120. In particular, the sterile treatment tool 128 is not enclosed with the sterile envelope 120.

In a method step 940, the enclosed handle 114 and the treatment tool 128 are releasably connected. In particular, the enclosed handle 114 and the treatment tool 128 are releasably connected by releasably connecting the proximal end 154 of the treatment tool 128 and the distal end 156 of the enclosed handle 114.

In a method step 950, the opening 144 of the sterile envelope 120 is sealingly closed.

Fig. 5 shows different tip portions 150a, 150b, 150c, 150d having different sizes and geometries, which, according to the present disclosure, can be used in combination with the same handle 114. The different tip portions 150a, 150b, 150c, 150d are straight or angled any may serve as pointers for cranial surgery. The surgeon may simply choose the appropriate tip portion 150a, 150b, 150c, 150d among the plurality of tip portions 150a, 150b, 150c, 150d to be plugged for a specific surgery.

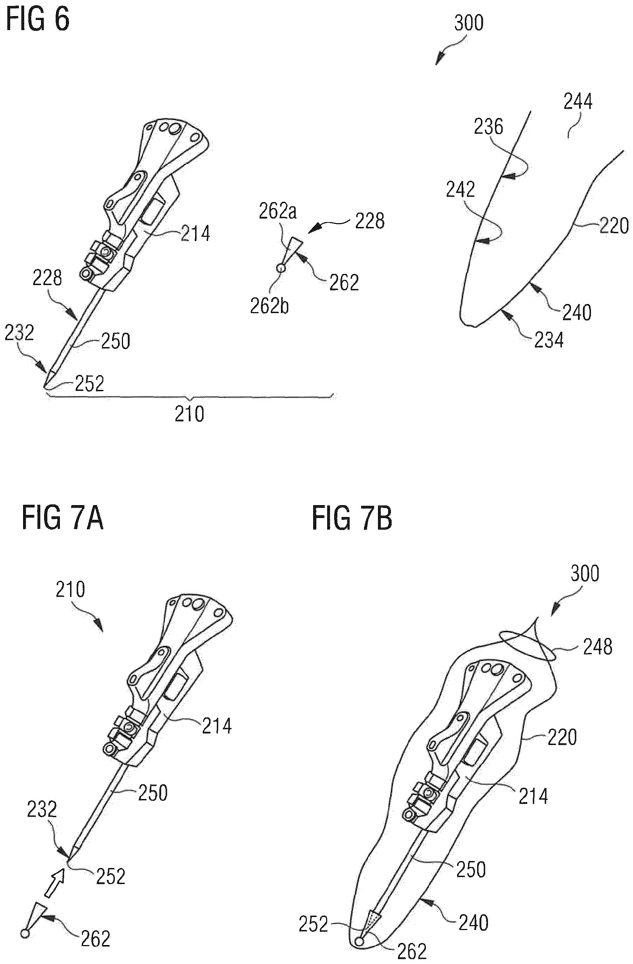

Fig. 6 shows the components of another example of a handheld instrument assembly 300 according to the present disclosure. The handheld instrument assembly 300 comprises a handheld instrument 210 and a sterile envelope 220. A blunt shaped cap 262 forms part of the handheld instrument 210. In particular, the blunt shaped cap 262 forms a releasable portion of the handheld instrument 210.

The handheld instrument 210 further comprises a non-sterile handle 214 and a non-sterile tip portion 250. The tip portion 250 is fixedly or releasably connected to the handle 214. In the present example, the tip portion 250 is cylindrically shaped and has a sharp tip 252 forming a distal end 232 of the tip portion 250. The tip portion 250 may be made of a metal.

The handle 214 is configured to be gripped by a user of the handheld instrument assembly 300 and comprises one or more tracking elements 218, in particular one or more active tracking elements 218. At least the handle 214 of the handheld instrument 300 is a re-usable component. In some implementations, also the tip portion 250 and/or the blunt shaped cap 262 are re-usable components.

The sterile envelope 220 may be configured as the sterile envelope 120 of the example of Figs. 3 and 4. The sterile envelope 220 may be a disposable item. In particular, the sterile envelope 220 comprises an outer surface side 240 that is a sterile surface side 234. In the present example, the inner surface side 242 is a non-sterile surface side 236. In other examples, the inner surface side 242 is also a sterile surface side 234.

The blunt shaped cap 262 is configured to be put on the sharp tip 252 of the tip portion 250. In the present example, the blunt shaped cap 262 is shaped like a hollow cone 262a with a rounded tip 262b. Furthermore, in the present example, the blunt shaped cap 262 is configured to be stuck on the distal end 232 of the tip portion 250. For that purpose, the blunt shaped cap 262 may be configured adhesive. For example, the blunt shaped cap 262 may be configured adhesive by providing an adhesive tape inside the hollow cone 262a or by providing an adhesive, for example a glue, inside the hollow cone 262a. The tip portion 250 and the blunt shaped cap 262 form part of the treatment tool 228. In particular, the blunt shaped cap 262 is a releasable portion of the treatment tool 228. The blunt shaped cap 262 is also non-sterile. As already discussed above, the blunt shaped cap 262 may be a re-usable item or a disposable item.

An assembly procedure for the handheld instrument assembly 300 of Fig. 6 is shown in Figs. 7A and 7B.

As illustrated in Fig. 7A, the handheld instrument 210 with the handle 214 and the tip portion 250 and the blunt shaped cap 262 is provided. The blunt shaped cap 262 is structurally separate and disconnected from the handle 214 with the tip portion 250. In the further assembly procedure, the blunt shaped cap 262 is stuck on the distal end 232 with the sharp tip 252 of the tip portion 250 thereby hiding the sharp tip 252 of the tip portion 250. Since the blunt shaped cap 262 is configured adhesive, the blunt shaped cap 262 is stuck on the tip portion 250 so as to be fixedly attached to the tip portion 250, i.e. to be secured in place relative to the tip portion 250. However, the connection may be released when needed and is not a permanent connection.

In a subsequent step shown in Fig. 7B, the sterile envelope 220 is provided. The sterile envelope 220 has at least an outer sterile surface side 34, 40. The sterile envelope 220 is pulled over the handheld instrument 210 with the blunt shaped cap 262 so as to completely receive and surround the handheld instrument 210 with the blunt shaped cap 262. In a last step, the sterile envelope 220 is sealingly closed, for example by an elastic 248 or an adhesive. In particular, the elastic 248 closes an opening 244 of the sterile envelope 220 to form a closed bag.

Due to the blunt shaped cap 262 that hides the sharp tip 252 of the tip portion 250, there is no risk that the sterile envelope 220 is punctured by the sharp tip 252 of the tip portion 250. Furthermore, since the sterile envelope 220 completely encloses the handheld instrument 210, sterility of the handheld instrument assembly 300 is ensured.

After surgery, the surgeon or the nurse may remove the sterile envelope 220 from the handheld instrument 210 and discard it. In some implementations, also the blunt shaped cap 262 is removed and discarded. In some implementations, also the tip portion 250 may be released from the handle 214. In these implementations, the tip portion 250 also may be a disposable.

For a subsequent surgery, the handheld instrument 210 with the handle 214, the tip portion 250 and the blunt shaped cap 262 is cleaned and disinfected and another, unused sterile envelope 220 may be provided.

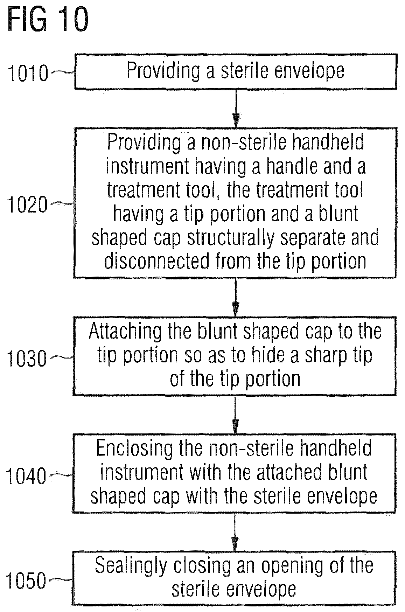

Fig. 10 of the drawings shows a flow chart schematically depicting the process steps of the method described with reference to Figs. 7A and 7B. In a method step 1010, a sterile envelope 220 is provided. The sterile envelope 220 has an opening 244 or is able to be formed to define an opening 244.

In a method step 1020, a non-sterile handheld instrument 210 having a handle 214 and a treatment tool 228 is provided. The treatment tool 228 has a tip portion 250 and a blunt shaped cap 262. The blunt shaped cap 262 is separate from and disconnected from the tip portion 250. The tip portion 250 extends from the handle 214 in a distal direction of the handheld instrument 210 and has a sharp tip 252 at a distal end 232.

In a method step 1030, the blunt shaped cap 262 is attached to the tip portion 250 so as to hide the sharp tip 252 of the tip portion 250.

In a method step 1040, the non-sterile handheld instrument 210 with the attached blunt shaped cap 262 is enclosed with the sterile envelope 220.

In a method step 1050, the opening 244 of the sterile envelope 220 is sealingly closed.

Generally, the above embodiments provide alternative solutions to the problem of providing sterile handheld instrument assemblies which have an increased lifetime and can be manufactured at low costs. Specifically, conventional sterile handheld instrument assemblies, if provided as single-use parts, would unduly raise the costs, and, if provided at re-usable parts that have to be exposed to a sterilization process such as autoclave, would decrease the lifetime and also increase the costs.

The above embodiments provide alternative solutions to the problem of providing sterile handheld instrument assemblies with sharp geometries, which have an increased lifetime and can be manufactured at low costs. The above embodiments also provides alternative solutions to the problem of providing sterile handheld instrument assemblies including one or more tracking elements which have an increased lifetime and can be manufactured at low costs.

Specifically, the above embodiments have in common that the handle 14, 114, 214 is enveloped with the sterile envelope 20, 120, 220 in order to provide sterility, and that features with sharp geometries 22, 150, 250 are either not covered by the sterile envelope 20, 120, 220 or are hidden by a blunt shaped cap 262. Tracking elements can also be covered with the sterile envelope. Thus, cost-intensive features and features that are sensitive to the sterilization process are covered by the sterile envelope and are reliably sealed by the sterile envelope, whereas less cost- intensive features may be provided as sterile components, either as re-usable or disposable items.

## Claims

1. A handheld instrument assembly (100, 200, 300) for use in a surgical environment, comprising
a non-sterile handle (14, 114, 214),
a treatment tool (28, 128, 228) extending from the handle (14, 114, 214) in a distal direction of the handheld instrument assembly (100, 200, 300), wherein the treatment tool (28, 128, 228) comprises a releasable portion (22, 150, 262) which is releasably attached or attachable to the handle (14, 114) or to an adjoining portion (16, 250) of the treatment tool (28, 128, 228), and
a sterile envelope (20, 120, 220) enveloping the non-sterile handle (14, 114, 214) for being gripped by a user of the handheld instrument assembly (100, 200, 300).

2. The assembly (100, 200, 300) of claim 1, comprising
one or more tracking elements (18, 118, 218), wherein the sterile envelope (20, 120, 220) is arranged to envelop the one or more tracking elements (18, 118, 218).

3. The assembly (100, 200, 300) of claim 2, wherein
the one or more tracking elements (18, 118, 218) comprise one or more active tracking elements (18, 118, 218), and wherein the sterile envelope (20, 120, 220) is transparent with regard to at least part of the spectrum of the light emitted by the active tracking elements (18, 118, 218).

4. The assembly (100) of any of the preceding claims, wherein
the releasable portion (22) forms a distal portion (26) of the treatment tool (28) and is releasably attached or attachable to a proximal portion (16) of the treatment tool (28), the proximal portion (16) of the treatment tool (28) being enveloped by the sterile envelope (20).

5. The assembly (100) of any of the preceding claims, wherein
the releasable portion (22) is fixed to or integral with the sterile envelope (20).

6. The assembly (200) of any of claims 1 to 3, wherein
the releasable portion (150) forms the treatment tool (128) which is releasably attached or attachable at a proximal end to the handle (114) enveloped by the sterile envelope (120), wherein the treatment tool (128) is not enveloped by the sterile envelope (120).

7. The assembly (100, 200) of any of the preceding claims, wherein
the releasable portion (22, 150) forms a sharp tip (52, 152) of the treatment tool (28).

8. The assembly (100, 200) of any of the preceding claims, wherein
the releasable portion (22, 150) is sterile.

9. The assembly (100, 200) of any of the preceding claims, wherein
the releasable portion (22, 150) is releasably attached or attachable by a plug connection.

10. The assembly (300) of any of claims 1 to 3, wherein
the releasable portion is a blunt shaped cap (262) hiding a sharp distal end (232, 252) of the treatment tool (250), the treatment tool (250) with the blunt shaped cap (262) being enveloped by the sterile envelope (220).

11. The assembly (100, 200, 300) of any of the preceding claims, wherein
the sterile envelope (20, 120, 220) has, or can be formed to define, an opening (44, 144, 244) for inserting the non-sterile handle (14, 114, 214) and, optionally, the treatment tool (128, 228) or a proximal portion (16) of the treatment tool (28), wherein the opening (44, 144, 244) is sealingly closed in an assembled state of the handheld instrument assembly (100, 200, 300).

12. The assembly (100, 200, 300) of any of the preceding claims, wherein
the sterile envelope (20, 120, 220) has an inner non-sterile surface side (36, 42, 136, 142, 236, 242) and an outer sterile surface side (34, 40, 134, 140, 234, 240), the outer sterile surface side (34, 40, 134, 140, 234, 240) for being gripped by the user of the handheld instrument assembly (100, 200, 300).

13. The assembly (100, 200, 300) of any of the preceding claims, wherein
at least one of the releasable portion (22, 150, 262) and the sterile envelope (20, 120, 220) is a disposable item.

14. The assembly (100, 200, 300) of any of the preceding claims, wherein
the handheld instrument assembly (100, 200, 300) is a navigation pointer.

15. A cover assembly (12) for use with a handheld instrument (10) in a surgical environment, comprising
a sterile envelope (20) having a sterile outer surface side (34, 40) for being gripped by a user of the handheld instrument (10), the sterile envelope (20) being configured to envelop the handheld instrument (10), and
a distal treatment portion (26) being fixed to or integral with the sterile envelope (20) so as to protrude from the sterile outer surface side (34, 40) of the sterile envelope (20), the distal treatment portion (26) being configured to be releasably attachable to a proximal treatment portion (16) or a handle (14) of the handheld instrument (10) so as to form a treatment tool (28).

16. The cover assembly (12) of claim 15, wherein
the distal treatment portion (26) of the envelope (20) comprises a sharp tip (52) configured to form a distal end (32) of the treatment tool (28).

17. A handheld instrument (10) for use in a surgical environment and for use with a cover assembly (12), the cover assembly (12) having a sterile envelope (20) being configured to envelop the handheld instrument (10), and a distal treatment portion (26) being fixed to or integral with the sterile envelope (20), the handheld instrument (10) comprising
a non-sterile handle (14),
a non-sterile proximal treatment portion (16) extending from the handle (14) in a distal direction of the handheld instrument (10),
wherein the proximal treatment portion (16) is configured to be releasably attachable to the distal treatment portion (26) of the cover assembly (12) so as to form a treatment tool (28).

18. A method of assembling a handheld instrument assembly for use in a surgical environment, comprising the steps of
- providing a cover assembly including a sterile envelope having, or being able to be formed to define, an opening and having a first, sterile envelope surface side, and a distal treatment portion being fixed to or integral with the sterile envelope so as to protrude from the first, sterile envelope surface side into a hollow space delimited by the first, sterile envelope surface side (810),
- providing a handheld instrument comprising a non-sterile handle and, optionally, a non-sterile proximal treatment portion extending from the handle in a distal direction of the handheld instrument (820),
- releasably connecting the cover assembly and the handheld instrument by releasably connecting a proximal end of the distal treatment portion of the cover assembly to a distal end of the non-sterile proximal treatment portion of the handheld instrument or to the handle of the handheld instrument, the proximal end of the distal treatment portion of the cover assembly being accessible from a second envelope surface side opposite the first, sterile envelope surface side (830), and
- after having established the releasable connection, everting the sterile envelope so that the first, sterile envelope surface side becomes an outer, sterile envelope surface side and the second envelope surface side becomes in inner surface side delimiting a hollow space receiving the non-sterile handle of the handheld instrument and, optionally, the non-sterile proximal treatment portion of the handheld instrument (840),
- sealingly closing the opening of the sterile envelope (850).

19. A method of assembling a handheld instrument assembly for use in a surgical environment, comprising the steps of
- providing a transparent sterile envelope having, or being able to be formed to define, an opening (910),
- providing a handheld instrument having a non-sterile handle with one or more tracking elements and a sterile treatment tool being structurally separate and disconnected from the non-sterile handle, wherein a proximal end of the treatment tool and a distal end of the handle are configured to be releasably attachable with each other (920),
- enclosing the non-sterile handle having the one or more tracking elements with the transparent sterile envelope (930),
- releasably connecting the enclosed handle and the treatment tool by releasably connecting the proximal end of the treatment tool and the distal end of the enclosed handle (940), and
- sealingly closing the opening of the sterile envelope (950).

20. A method of assembling a handheld instrument assembly for use in a surgical environment, comprising the steps of
- providing a sterile envelope having, or being able to be formed to define, an opening (1010),
- providing a non-sterile handheld instrument having a handle and a treatment tool, the treatment tool having a tip portion extending from the handle in a distal direction of the handheld instrument and having a blunt shaped cap structurally separate from and disconnected from the tip portion, the tip portion having a sharp tip at a distal end (1020),
- attaching the blunt shaped cap to the distal end of the tip portion so as to hide the sharp tip (1030),
- enclosing the non-sterile handheld instrument with the attached blunt shaped cap with the sterile envelope (1040), and
- sealingly closing the opening of the sterile envelope (1050).

## Amended claims

### Amended claims in accordance with Rule 137(2) EPC.

1. A handheld instrument assembly (100, 200, 300) for use in a surgical environment, comprising
a non-sterile handle (14, 114, 214),
a treatment tool (28, 128, 228) extending from the handle (14, 114, 214) in a distal direction of the handheld instrument assembly (100, 200, 300), wherein the treatment tool (28, 128, 228) comprises a releasable portion (22, 150, 262) which is releasably attached or attachable to the handle (14, 114) or to an adjoining portion (16, 250) of the treatment tool (28, 128, 228),
one or more active tracking elements (18, 118, 218) configured to emit light, and
a sterile envelope (20, 120, 220) enveloping the non-sterile handle (14, 114, 214) for being gripped by a user of the handheld instrument assembly (100, 200, 300) and enveloping the one or more active tracking elements (18, 118, 218),
wherein the sterile envelope (20, 120, 220) is flexible so as to closely hug the contour of the non-sterile handle (14, 114, 214) and the one or more active tracking elements (18, 118, 218), and wherein the sterile envelope (20, 120, 220) is transparent with regard to at least part of the spectrum of the light emitted by the active tracking elements (18, 118, 218).

2. The assembly (100) of claim 1, wherein
the releasable portion (22) forms a distal portion (26) of the treatment tool (28) and is releasably attached or attachable to a proximal portion (16) of the treatment tool (28), the proximal portion (16) of the treatment tool (28) being enveloped by the sterile envelope (20).

3. The assembly (100) of any of the preceding claims, wherein
the releasable portion (22) is fixed to or integral with the sterile envelope (20).

4. The assembly (200) of claim 1, wherein
the releasable portion (150) forms the treatment tool (128) which is releasably attached or attachable at a proximal end to the handle (114) enveloped by the sterile envelope (120), wherein the treatment tool (128) is not enveloped by the sterile envelope (120).

5. The assembly (100, 200) of any of the preceding claims, wherein
the releasable portion (22, 150) forms a sharp tip (52, 152) of the treatment tool (28).

6. The assembly (100, 200) of any of the preceding claims, wherein
the releasable portion (22, 150) is sterile.

7. The assembly (100, 200) of any of the preceding claims, wherein
the releasable portion (22, 150) is releasably attached or attachable by a plug connection.

8. The assembly (300) of claim 1, wherein
the releasable portion is a blunt shaped cap (262) hiding a sharp distal end (232, 252) of the treatment tool (250), the treatment tool (250) with the blunt shaped cap (262) being enveloped by the sterile envelope (220).

9. The assembly (100, 200, 300) of any of the preceding claims, wherein
the sterile envelope (20, 120, 220) has, or can be formed to define, an opening (44, 144, 244) for inserting the non-sterile handle (14, 114, 214) and, optionally, the treatment tool (128, 228) or a proximal portion (16) of the treatment tool (28), wherein the opening (44, 144, 244) is sealingly closed in an assembled state of the handheld instrument assembly (100, 200, 300).

10. The assembly (100, 200, 300) of any of the preceding claims, wherein
the sterile envelope (20, 120, 220) has an inner non-sterile surface side (36, 42, 136, 142, 236, 242) and an outer sterile surface side (34, 40, 134, 140, 234, 240), the outer sterile surface side (34, 40, 134, 140, 234, 240) for being gripped by the user of the handheld instrument assembly (100, 200, 300).

11. The assembly (100, 200, 300) of any of the preceding claims, wherein
at least one of the releasable portion (22, 150, 262) and the sterile envelope (20, 120, 220) is a disposable item.

12. The assembly (100, 200, 300) of any of the preceding claims, wherein
the handheld instrument assembly (100, 200, 300) is a navigation pointer.

13. A method of assembling a handheld instrument assembly for use in a surgical environment, comprising the steps of
- providing a flexible sterile envelope having, or being able to be formed to define, an opening and being at least partially transparent with respect to a spectrum of light emitted by one or more active tracking elements of a handheld instrument (810, 910, 1010),
- providing the handheld instrument comprising a non-sterile handle with one or more active tracking elements configured to emit light and a treatment tool, wherein the treatment tool comprises a releasable portion which is releasably attached or attachable to the non-sterile handle or to an adjoining portion of the treatment tool (820, 920, 1020),
- enclosing the non-sterile handle having the one or more active tracking elements with the transparent flexible sterile envelope so as to closely hug the contour of the non-sterile handle with the one or more active tracking elements (840, 930, 1040), and
- sealingly closing the opening of the sterile envelope (850, 950, 1050).

14. The method of claim 13, wherein the transparent flexible sterile envelope has a first, flexible sterile envelope surface side being fixed to or integral with a distal treatment portion of the treatment tool forming the releasable portion of the treatment tool, the distal treatment portion protruding from the first, flexible sterile envelope surface side into a hollow space delimited by the first, flexible sterile envelope surface side, the transparent flexible sterile envelope and the distal treatment portion forming a cover assembly, the method further comprising
- releasably connecting the cover assembly and the handheld instrument by releasably connecting a proximal end of the distal treatment portion of the cover assembly to a distal end of a non-sterile proximal treatment portion of the handheld instrument or to the handle of the handheld instrument, the proximal end of the distal treatment portion being accessible from a second envelope surface side opposite the first sterile envelope surface side (830), and
- after having established the releasable connection, performing the step of enclosing the non-sterile handle with the transparent flexible sterile envelope by everting the flexible sterile envelope so that the first, flexible sterile envelope surface side becomes an outer, flexible sterile envelope surface side and the second flexible envelope surface side becomes an inner surface side delimiting a hollow space receiving the non-sterile handle with the one or more active tracking elements and, optionally, the non-sterile proximal treatment portion of the handheld instrument (840).

15. The method of claim 13, wherein the treatment tool is a sterile treatment tool structurally separate and disconnected from the non-sterile handle, wherein a proximal end of the treatment tool and a distal end of the handle are configured to be releasably attachable with each other, the method further comprising the step of
- after having enclosed the non-sterile handle with the transparent flexible sterile envelope, releasably connecting the enclosed handle and the treatment tool by releasably connecting the proximal end of the treatment tool and the distal end of the enclosed handle (940).

16. The method of claim 13, wherein the treatment tool has a tip portion extending from the handle in a distal direction of the handheld instrument and a blunt shaped cap structurally separate from and disconnected from the tip portion, the tip portion having a sharp tip at a distal end, the method further comprising the steps
- attaching the blunt shaped cap to the distal end of the tip portion so as to hide the sharp tip (1030), and
- enclosing the non-sterile handheld instrument with the attached blunt shaped cap with the flexible sterile envelope (1040).
